# EUROPEAN PATENT APPLICATION

(11) **EP 1 992 326 A2**
(43) Date of publication of application: **19.11.2008**
(21) Application number: 08009140.8
(22) Date of filing: 16.05.2008
(51) Int. Cl.: A61K 8/11, A61K 8/64, A61Q 19/00

(54) **External dermatologic preparation**

(30) Priority: 16.05.2007 JP 2007130503
(71) Applicant: FUJIFILM Corporation, Minato-ku Tokyo (JP)
(72) Inventor: Ogiwara, Kazutaka, Ashigarakami-gun Kanagawa 258-8538 (JP); Aimi, Makiko, Ashigarakami-gun Kanagawa 258-8538 (JP); Ooya, Shouji, Ashigarakami-gun Kanagawa 258-8538 (JP)
(74) Representative: HOFFMANN EITLE

(57) **Abstract**

An object of the present invention is to provide an external dermatologic preparation which comprises nanoparticles consisting of proteins that can exhibit desirable effects via regulation of particle diameters. The present invention provides an external dermatologic preparation which comprises protein nanoparticles containing an active ingredient and having an average particle size of 200 to 500 nm.

## Description

### Technical Field

The present invention relates to an external dermatologic preparation which comprises protein nanoparticles containing an active ingredients and having an average particle size of 200 to 500 nm.

### Background Art

The skin absorbs external stimuli and regulates loss of components within the body such as a moisture, and the epidermis is at the forefront of such activities. The epidermis is composed of a stratum basale, a stratum spinosum, a stratum granulosum, and a stratum corneum. Cells that had undergone cell division and growth in the stratum basale repeat skin turnover via differentiation while passing through the stratum spinosum and the stratum granulosum, formation of a stratum corneum composed of strongly crosslinked keratin protein fibers, and desquamation at the final stage.

On an epidermis that has not experienced normal turnover, differentiation of epidermal keratinocytes, which should take place during the turnover process, takes place abnormally. Production of skin moisturizing factors that are produced via normal differentiation, such as NMFs, and lipids between cornified cells such as ceramides, becomes abnormal. Original skin functions are lost, which results in skin problems, such as surface dryness, dry skin, or rough skin. In recent years, stimuli such as a dry atmosphere, ultraviolet rays, and stress enhance proliferation of epidermal keratinocytes. Such stimuli also cause deterioration of stratum corneum barrier functions resulting from immature cornified cells due to the lack of cornification of normal epidermal keratinocytes, which would often cause surface dryness, dry skin, and rough skin.

As means for alleviating surface dryness, dry skin, and rough skin resulting from abnormal turnover of skin cells, external dermatologic preparations, cosmetic lotions, and the like comprising various types of moisturizing agents have been heretofore mainly used. These means provide moisture to the skin to improve skin feel temporarily; however, such means would not produce satisfactory effects of improving conditions of surface dryness, dry skin, and rough skin.

In recent years, it has been desired that cosmetic products show more distinct effects on the skin. Improvement in functionality and usability and differentiation of a given product from products of other companies have been attempted via employment of various new technologies, such as nanotechnology. For example, nanotechnology-based drug delivery systems (DDSs) for the purpose of improving skin permeability have drawn attention, and skin permeability improved by nanoparticulation of active ingredients has been reported (see, for example, JP Patent Publication (kokai) No. 2002-308728 A). However, organic solvents or surfactants, which are synthetic polymers, used for nanoparticulation of active ingredients are irritating, and such substances are problematic in terms of safety, since the skin of a patient sensitive to a lower alcohol may sensitively react to such substances, and the skin may be damaged by ethanol or surfactants or excessively stimulated (see, for example, Li Lingna et al., PNAS 88, 1908-1912, 1991). Also, commercialized cosmetic products often comprise active ingredients dissolved therein with the aid of organic solvents (ethanol, in general) or surfactants. This sometimes causes trouble on skin, such as roughening or exanthem.

Meanwhile, naturally-occurring polymers are derived from organisms that exist in nature. Examples of naturally-occurring polymers include hydrophilic proteins or polysaccharides derived from plants or animals and polysaccharides produced by microorganisms. Since such substances are naturally occurring, and such substances are often used in the food, pharmaceutical, and cosmetic industries, which require safety. While naturally-occurring polymers exhibit high structural stability, as do synthetic polymers, naturally-occurring polymers have remarkably high safety compared with synthetic polymers, and naturally-occurring polymers by themselves advantageously have physiological activity such as moisturizing activity. Particles that comprise naturally-occurring polymers such as casein having moisturizing effects as carriers are mostly micron-sized, and size regulation thereof is thus difficult (see, for example, JP Patent Publication (kohyo) No. 2005-500304 (A)).

### Disclosure of the Invention

An object of the present invention is to overcome the above-described problems of conventional techniques. More particularly, an object of the present invention is to provide an external dermatologic preparation which comprises nanoparticles consisting of proteins that can exhibit desirable effects via regulation of particle diameters.

The present inventors have conducted concentrated studies in order to attain the above object. They prepared protein nanoparticles containing an active ingredient and having an average particle size of 200 to 500 nm, and they applied such nanoparticles to the skin. As a result, they discovered that satisfactory effects could be attained by particles remaining in the stratum corneum. Such nanoparticles are highly safe and translucent The present invention has been completed based on these findings.

Specifically, the present invention provides an external dermatologic preparation which comprises protein nanoparticles containing an active ingredient and having an average particle size of 200 to 500 nm.

Preferably, the external dermatologic preparation of the present invention contains protein nanoparticles in an amount of 1% to 50% by weight.

Preferably, the external dermatologic preparation of the present invention contains an active ingredient in a weight that is 0.1 % to 100% of the protein weight.

Preferably, the active ingredient is at least one selected from the group consisting of ultraviolet absorber or moisturizing component.

Preferably, the active ingredient is an ionic substance or a fat-soluble substance.

Preferably, the protein is at least one selected from the group consisting of collagen, gelatin, acid-treated gelatin, albumin, ovalbumin, casein, transferrin, globulin, fibroin, fibrin, laminin, fibronectin, and vitronectin.

Preferably, the protein is subjected to crosslinking treatment during and/or after nanoparticle formation.

Preferably, crosslinking treatment is carried out by an enzyme.

The enzyme is not particularly limited so long as it has a function of crosslinking a protein, and preferably transglutaminase is used.

Preferably, the external dermatologic preparation of the present invention comprises casein nanoparticles prepared by the following steps (a) to (c):
(a) mixing casein with a basic aqueous medium at pH of from 8 to less than 11;
(b) adding at least one active ingredient to the solution obtained in step (a); and
(c) injecting the solution obtained in step (b) into an aqueous medium at pH of 3.5 to 7.5:

Preferably, the external dermatologic preparation of the present invention comprises casein nanoparticles prepared by the following steps (a) to (c):
(a) mixing casein with a basic aqueous medium at pH of from 8 to less than 11;
(b) adding at least one active ingredient to the solution obtained in step (a); and
(c) lowering the pH of the solution obtained in step (b) to pH value which is different from the isoelectric point by 1 or more units.

Particles containing an active ingredient in the external dermatologic preparation of the present invention are nanoparticles having particle diameters regulated at 200 to 500 nm on average. Thus, such nanoparticles remain in the stratum corneum and are highly functional. Because of the use of protein nanoparticles, the external dermatologic preparation can be produced without the use of a chemical crosslinking agent or synthetic surfactant. Thus, the resulting preparation is highly safe.

### Best Modes for Carrying out the Invention

Hereafter, the embodiments of the present invention are described in greater detail.

The external dermatologic preparation of the present invention comprises protein nanoparticles containing an active ingredient and having an average particle size of 200 to 500 nm.

Active ingredients to be used in the present invention are not particularly limited, provided that such ingredients are absorbed in the stratum corneum, remain therein, and exhibit activity. Preferably, the active ingredient can be selected from ultraviolet absorbers or moisturizing components. Examples of ultraviolet absorbers for cosmetic products that have been used in the past include benzoic acid derivatives, cinnamic acid derivatives, benzophenone derivatives, dibenzoylmethane derivatives, and salicylic acid derivatives, classified according to structure.

Examples of moisturizing components include Moutan cortex extract, Hydrangeae dulcis folium extract, hypericum extract, coleus extract, Euonymus japonica, safflower extract, Rosa rugosa flower extract, Polyporus Sclerotium extract, hawthorn extract, rosemary extract, duku extract, chamomile extract, lamium album extract, Litchi Chinensis extract, Achillea Millefolium extract, aloe extract, marronnier extract, Thujopsis dolabrata extract, Fucus extract, Osmoin extract, oat extract, Tuberosa polysaccharide, Cordyceps Sinensis extract, barley extract, orange extract, Rehmannia root extract, zanthoxylum fruit extract, and coix seed extract. Such active ingredient may be used alone or in combinations of two or more. Since casein by itself has moisturizing effects, nanoparticles containingno such moisturizing components are also preferable. Also, it was discovered that active substances could be included in casein nanoparticles with the aid of interactions between fat-soluble active substances and casein hydrophobic portions, and that such particles were present stably in aqueous solutions. Thus, an active substance having ClogP of greater than 0 is preferable. ClogP of 1 or greater is more preferable, and ClogP of 3 or greater is further preferable.

Further, it was found that a particle mixture of casein and ionic polysaccharide or another ionic protein can contain an ionic active ingredient.

The external dermatologic preparation of the present invention comprises preferably 0.01% to 50% by weight and most preferably 0.1% to 10% by weight protein nanoparticles.

The external dermatologic preparation of the present invention contains an active ingredient in a weight that is preferably 0.1% to 100% and more preferably 0.1% to 50% of the protein weight.

According to the present invention, an active ingredient may be added during or after protein nanoparticle formation.

The average particle size of protein nanoparticles used in the present invention is generally 200 to 500 nm, preferably 200 to 300 nm.

The type of protein used in the present invention is not particularly limited. However, a protein having a lysine residue and a glutamine residue is preferable. In addition, such protein having a molecular weight of approximately 10,000 to 1,000,000 is preferably used. The origin of the protein is not particularly limited. However, a naturally occurring protein is preferably used, and a human-derived protein is particularly preferably used. Specific examples of a protein that can be used may include at least one selected from the group consisting of collagen, gelatin, acid-treated gelatin, albumin, ovalbumin, casein, transferrin, globulin, fibroin, fibrin, laminin, fibronectin, and vitronectin. However, the compound used in the present invention is not limited to the aforementioned compounds. In addition, the origin of the protein is not particularly limited. Thus, bovine, swine, fish or plant protein, as well as recombinant protein of any thereof, can be used. Examples of recombinant gelatin that can be used include, but are not limited to, gelatins described in EP1014176 A2 and US Patent No. 6,992,172. Among them, casein, acid-treated gelatin, collagen, or albumin is preferable. Further, casein or acid-treated gelatin is most preferable. When casein is used in the present invention, the origin of the casein is not particularly limited. Casein may be milk-derived or bean-derived. Any of α-casein, β-casein, γ-casein, and κ-casein, as well as a mixture thereof, can be used. Caseins may be used alone or in combinations of two or more.

Proteins used in the present invention may be used alone or in combinations of two or more.

According to the present invention, a protein can be subjected to crosslinking treatment during and/or after nanoparticle formation. For the crosslinking treatment, an enzyme can be used. Any enzyme may be used without particular limitation as long as it has been known to have an action of causing protein crosslinking. Among such enzymes, transglutaminase is preferable.

Transglutaminase may be derived from a mammal or a microorganism. A recombinant transglutaminase can be used. Specific examples thereof include the Activa series by Ajinomoto Co., Inc., commercially available mammalian-derived transglutaminase serving as a reagent, such as guinea pig liver-derived transglutaminase, goat-derived transglutaminase, rabbit-derived transglutaminase, or human-derived recombinant transglutaminase produced by, for example, Oriental Yeast Co., Ltd., Upstate USA Inc., and Biodesign International.

The amount of an enzyme used for the crosslinking treatment in the present invention can be adequately determined depending upon protein type. In general, an enzyme can be added in a weight that is 0.1% to 100% and preferably approximately 1% to 50% of the protein weight.

The duration for an enzymatic crosslinking reaction can be adequately determined depending upon protein type and nanoparticle size. However, in general, the reaction can be carried out for 1 to 72 hours, and preferably 2 to 24 hours.

The temperature for an enzymatic crosslinking reaction can be adequately determined depending upon protein type and nanoparticle size. In general, the reaction can be earned out at 0°C to 80°C and preferably at 25°C to 60°C.

Enzymes used in the present invention may be used alone or in combinations of two or more.

Nanoparticles of the present invention can be prepared in accordance with Patent Document: JP Patent Publication (Kokai) No. 6-79168 A (1994); or C. Coester, Journal Microcapsulation, 2000, vol. 17, pp. 187-193, provided that an enzyme is preferably used instead of glutaraldehyde for a crosslinking method.

In addition, according to the present invention, the enzymatic crosslinking treatment is preferably carried out in an organic solvent. The organic solvent used herein is preferably an aqueous organic solvent such as ethanol, isopropanol, acetone, or THF.

Further, according to the present invention, it is preferable to remove an organic solvent by distillation subsequent to a crosslinking treatment, followed by water dispersion. It is also possible to add water prior to or subsequent to removal of an organic solvent by distillation.

It is also possible to add at least one component selected from the group consisting of lipids (e.g., phospholipid), anionic polysaccharides, cationic polysaccharides, anionic proteins, cationic proteins, and cyclodextrin to the external dermatologic preparation of the present invention. The amounts of lipid (e.g. phospholipid), anionic polysaccharide, cationic polysaccharide, anionic protein, cationic protein, and cyclodextrin to be added are not particularly limited. However, they can be added usually in a weight that is 0.1% to 100% of the protein weight. In the case of the external dermatologic preparation of the present invention, it is possible to adjust the release rate by changing the ratio of the above components to the protein.

Specific examples of phospholipids that can be used in the present invention include, but are not limited to, the following compounds: phosphatidylcholine (lecithin), phosphatidylethanolamine, phosphatidylserine, phosphatidylinositol, phosphatidylglycerol, diphosphatidylglycerol, and sphingomyelin.

Anionic polysaccharides that can be used in the present invention are polysaccharides having an acidic polar group such as a carboxyl group, a sulfate group, or a phosphate group. Specific examples thereof include, but are not limited to, the following compounds: chondroitin sulfate, dextran sulfate, carboxymethyl cellulose, carboxymethyl dextran, alginic acid, pectin, carrageenan, fucoidan, agaropectin, porphyran, karaya gum, gellan gum, xanthan gum, and hyaluronic acids.

Cationic polysaccharides that can be used in the present invention are polysaccharides having a basic polar group such as an amino group. Examples thereof include, but are not limited to, the following compounds : polysaccharides such as chitin or chitosan, which comprise, as a monosaccharide unit, glucosamine or galactosamine.

Anionic proteins that can be used in the present invention are proteins and lipoproteins having a more basic isoelectric point than the physiological pH. Specific examples thereof include, but are not limited to, the following compounds: polyglutamic acid, polyaspartic acid, lysozyme, cytochrome C, ribonuclease, trypsinogen, chymotrypsinogen, and α-chymotrypsin.

Cationic proteins that can be used in the present invention are proteins and lipoproteins having a more acidic isoelectric point than the physiological pH. Specific examples thereof include, but are not limited to, the following compounds: polylysine, polyarginine, histone, protamine, and ovalbumin.

According to the present invention, it is possible to use casein nanoparticles prepared by the following steps (a) to (c) of:
(a) mixing casein with a basic aqueous medium at pH of 8 to less than 11;
(b) adding at least one active ingredient to the solution obtained in step (a); and
(c) injecting the solution obtained in step (b) into an aqueous medium at a pH of 3.5 to 7.5.

Further, according to the present invention, it is possible to use casein nanoparticles prepared by the following steps (a) to (c) of:
(a) mixing casein with a basic aqueous medium at a pH of 8 to less than 11;
(b) adding at least one active ingredient to the solution obtained in step (a); and
(c) lowering the pH of the solution obtained in step (b) to a pH value which is distinct from the isoelectric point by 1 unit or more.

In the present invention, with the use of interaction between a hydrophobic active ingredient and a casein hydrophobic domain, it is possible for casein nanoparticles to contain the active ingredient. In addition, it was found that such particles remain stable in an aqueous solution. Further, it was found that a particle mixture of casein and ionic polysaccharide or another ionic protein contains an ionic active ingredient.

The method for preparing casein nanoparticles of the present invention involves a method wherein casein is mixed with a basic aqueous medium solution and the solution is injected into another acidic aqueous medium, and a method wherein casein is mixed with a basic aqueous medium solution and the pH of the solution is lowered during stirring, for example.

The method wherein casein is mixed with a basic aqueous medium solution and the solution is injected into another acidic aqueous medium is preferably carried out using a syringe for convenience. However, there is no particular limitation as long as the injection rate, solubility, temperature, and stirring conditions are satisfied. Injection can be carried out usually at an injection rate of 1 mL/min to 100 mL/min. The temperature of the basic aqueous medium can be adequately determined. In general, the temperature is 0°C to 80°C and preferably 25°C to 70°C. The temperature of an aqueous medium can be adequately determined. In general, the temperature can be 0°C to 80°C and preferably 25°C to 60°C. The stirring rate can be adequately determined. However, in general, the stirring rate can be 100 rpm to 3000 rpm and preferably 200 rpm to 2000 rpm.

In the method wherein casein is mixed with a basic aqueous medium solution and the pH of the medium is lowered during stirring, it is preferable to add acid dropwise for convenience. However, there is no particular limitation as long as solubility, temperature, and stirring conditions are satisfied. The temperature of a basic aqueous medium can be adequately determined. However, in general, the temperature can be 0°C to 80°C and preferably 25°C to 70°C. The stirring rate can be adequately determined, However, in general, the stirring rate can be 100 rpm to 3000 rpm and preferably 200 rpm to 2000 rpm.

The aqueous medium that can be used for the present invention is an aqueous solution or a buffer comprising an organic acid or base or an inorganic acid or base.

Specific examples thereof include, but are not limited to, aqueous solutions comprising: organic acids such as citric acid, ascorbic acid, gluconic acid, carboxylic acid, tartaric acid, succinic acid, acetic acid, phthalic acid, trifluoroacetic acid, morpholinoethanesulfonic acid, and 2-[4-(2-hydroxyethyl)-1-piperazinyl]ethanesulfouic acid; organic bases such as tris (hydroxymethyl), aminomethane, and ammonia; inorganic acids such as hydrochloric acid, perchloric acid, and carbonic acid; and inorganic bases such as sodium phosphate, potassium phosphate, calcium hydroxide, sodium hydroxide, potassium hydroxide, and magnesium hydroxide.

The concentration of an aqueous medium used in the present invention is preferably approximately 10 mM to 1 M, and more preferably approximately 20 mM to 200 mM.

The pH of a basic aqueous medium used in the present invention is preferably 8 or more, more preferably 8 to 12, and further preferably 9 to 11. When the pH is excessively high, there is concern regarding hydrolysis or risks in handling. Thus, the pH is preferably in the above range.

According to the present invention, the temperature at which casein is mixed with a basic aqueous medium at pH of 8 or more is preferably 0°C to 80°C, more preferably 10°C to 60°C, and further preferably 20°C to 40°C.

The pH of an acidic aqueous medium used in the present invention is preferably 3.5 to 7.5 and more preferably 5 to 6.

The external dermatologic preparation of the present invention can further comprise additives. Additives are not particularly limited. Additives that can be used include at least one member selected from among moisturizing agents, softening agents, transdermal absorption enhancers, soothing agents, preservatives, antioxidants, coloring agents, thickeners, aroma chemicals, and pH adjusters. In order to impart desirable effects to the external dermatologic preparation of the present invention, protein nanoparticles having different particle diameters can also be mixed. For example, protein nanoparticles having an average particle size of less than 200 nm may be mixed, so that active ingredients that exhibit the effects in the stratum corneum and active ingredients that function in deeper parts of the skin can function simultaneously. Active ingredients described in, for example, JP Patent Application No. 2007-013261 can be used.

Specific examples of moisturizing agents that can be used in the present invention include, but are not limited to, the following compounds: agar, diglycerin, distearyldimonium hectorite, butylene glycol, polyethylene glycol, propylene glycol, hexylene glycol, *Coix lachrma-jobi* extract, vaseline, urea, hyaluronic acid, ceramide, Lipidure, isoflavone, amino acid, collagen, mucopolysaccharide, fucoidan, lactoferrin, sorbitol, chitin/chitosan, malic acid, glucuronic acid, placenta extract, seaweed extract, moutan cortex extract, sweet tea extract, hypericum extract, coleus extract, *Euonymus japonicus* extract, safflower extract, Rosa rugosa flower extract, *Polyporus sclerotium* extract, hawthorn extract, rosemary extract, duke extract, chamomile extract, *Lamium album* extract, *Litchi Chinensis* extract, *Achillea millefolium* extract, aloe extract, marronnier extract, *Thujopsis dolabrata* extract, Fucus extract, Osmoin extract, oat bran extract, tuberosa polysaccharide, *Cordyceps sinensis* (plant worm) extract, barley extract, orange extract, *Rehmannia glutinosa* extract, zanthoxylumb extract, and *Coix lachrma-jobi* extract.

Specific examples of softening agents that can be used in the present invention include, but are not limited to, the following compounds: glycerin, mineral oil, and emollient ingredients (e.g., isopropyl isostearate, polyglyceryl isostearate, isotridecyl isononanoate, octyl isononanoate, oleic acid, glyceryl oleate, cocoa butter, cholesterol, mixed fatty acid triglyceride, dioctyl succinate, sucrose tetrastearate triacetate, cyclopentasiloxane, sucrose distearate, palmitateoctyl, octyl hydroxystearate, arachidyl behenate, sucrose polybehenate, polymethylsilsesquioxane, myristyl alcohol, cetyl myristate, myristyl myristate, and hexyl laurate).

Specific examples of transdermal absorption enhancers that can be used in the present invention include, but are not limited to, the following compounds: ethanol, isopropyl myristate, citric acid, squalane, oleic acid, menthol, N-mcthyl-2-pyrrolidone, diethyl adipate, diisopropyl adipate, diethyl sebacate, diisopropyl sebacate, isopropyl palmitate, oleic acid isopropyl, oleic acid octyldodecyl, isostearyl alcohol, 2-octyldodecanol, urea, vegetable oil, and animal oil.

Specific examples of soothing agents that can be used in the present invention include, but are not limited to, the following compounds : benzyl alcohol, procaine hydrochloride, xylocaine hydrochloride, and chlorobutanol.

Specific examples of preservatives that can be used in the present invention include, but are not limited to, the following compounds : benzoic acid, sodium benzoate, paraben, ethylparaben, methylparaben, propylparaben, butylparaben, potassium sorbate, sodium sorbate, sorbic acid, sodium dehydroacetate, hydrogen peroxide, formic acid, ethyl formate, sodium hypochlorite, propionic acid, sodium propionate, calcium propionate, pectin degradation products, polylysine, phenol, isopropylmethyl phenol, orthophenylphenol, phenoxyethanol, resorcin, thymol, thiram, and tea tree oil.

Specific examples of antioxidants that can be used in the present invention include, but are not limited to, the following compounds: vitamin A, retinoic acid, retinol, retinol acetate, retinol palmitate, retinyl acetate, retinyl palmitate, tocopheryl retinoate, vitamin C and derivatives thereof, kinetin, β-carotene, astaxanthin, lutein, lycopene, tretinoin, vitamin E, α-lipoic acid, coenzyme Q10, polyphenol, SOD, and phytic acid.

Specific examples of coloring agents that can be used in the present invention include, but are not limited to, the following compounds: krill pigment, orange dye, cacao dye, kaoline, carmines, ultramarine blue, cochineal dye, chrome oxide, iron oxide, titanium dioxide, tar dye, and chlorophyll.

Specific examples of thickeners that can be used in the present invention include, but are not limited to, the following compounds: quince seed, carrageenan, gum arabic, karaya gum, xanthan gum, gellan gum, tamarind gum, locust bean gum, gum traganth, pectin, starch, cyclodextrin, methylcellulose, ethylcellulose, carboxymethylcellulose, sodium alginate, polyvinyl alcohol, polyvinyl pyrrolidone, carboxyvinyl polymer, and sodium polyacrylate.

Specific examples of aroma chemicals that can be used in the present invention include, but are not limited to, the following compounds: musk, acacia oil, anise oil, ylang ylang oil, cinnamon oil, jasmine oil, sweet orange oil, spearmint oil, geranium oil, thyme oil, neroli oil, mentha oil, hinoki (Japanese cypress) oil, fennel oil, peppermint oil, bergamot oil, lime oil, lavender oil, lemon oil, lemongrass oil, rose oil, rosewood oil, anisaldehyde, geraniol, citral, civetone, muscone, limonene, and vanillin.

Specific examples of pH adjusters that can be used in the present invention include, but are not limited to, the following compounds: sodium citrate, sodium acetate, sodium hydroxide, potassium hydroxide, phosphoric acid, and succinic acid.

Forms of the external dermatologic preparations of the present invention are not particularly limited. Examples of such forms include liquids, compresses, embrocations, gels, creams, aerosols, lotions, powders, foams, skin toning lotions, body washes, soaps, and cosmetics.

Specific examples of administration method of the external dermatologic preparations of the present invention may include transdermal administration and transmucosal administration.

The dose of the external dermatologic preparations of the present invention can be adequately determined depending upon type and amount of active ingredient and upon user weight and condition, for example. The dose for single administration is generally approximately 1 µg to 50 mg/cm² and preferably 2.5 µg to 10 mg/cm².

The present invention is hereafter described in greater detail with reference to the following examples, although the technical scope of the present invention is not limited thereto.

### Examples

### Example 1

Acid-treated gelatin (10 mg) and TG-S (5 mg, Ajinomoto Co.) were dissolved in 1 ml of water. The resulting gelatin solution (1 ml) was injected into 10 ml of ethanol comprising 1.7 mg of glycyrrhetic acid dissolved therein using a microsyringe with agitation at 800 rpm at a preset external temperature of 40°C, and gelatin nanoparticles were obtained. The resulting dispersion was allowed to stand at a preset external temperature of 55°C for 5 hours, and gelatin nanoparticles were subjected to crosslinking. Water (5 ml) was added to the resulting dispersion of gelatin nanoparticles, ethanol was removed using a rotary evaporator, and a dispersion of gelatin nanoparticles comprising glycyrrhetic acid therein in water was obtained. The average particle diameter of such particles was determined to be 201 nm as a result of measurement using a light scattering photometer (Microtrack, Nikkiso, Co., Ltd.).

### Example 2

Casein (100 mg, milk-derived, Wako Pure Chemical Industries, Ltd.) was mixed with 10 ml of 50 mM phosphate buffer (pH 10). Glycyrrhetic acid (5 mg) was dissolved in 0.1 ml of ethanol. These two solutions were mixed with each other, sodium hydroxide was added to the resulting solution to adjust the pH level at 10, and thus casein nanoparticles were obtained. The average particle diameter of such particles was determined to be 250 nm as a result of measurement using a light scattering photometer (Nanotrack, Nikkiso, Co., Ltd.).

### Test Example 1

The dispersions of nanoparticles described in Examples 1 and 2 were stored at room temperature for 1 month, and the average particle diameter of such particles was measured using Nikkiso's Microtrack.

As Comparative Example 1, Nanoimpact (Hosokawa Micron Group), which is a dispersion of nanoparticles of synthetic polymers (PLGA), was used.

The results of measurement in Test Example 1 are shown in Table 1.

**Table 1: Test Example 1**

| Appearance | Comparative Example 1 | Example 1 | Example 2 |
|---|---|---|---|
| At the time of adjustment | 600nm | 201 nm | 250 nm |
| 1 month later | N.D. | 222 nm | 274 nm |

| | | | |
|---|---|---|---|
| N.D.: not measurable | | | |

The above examples indicate that the external dermatologic preparation of the present invention is highly stable. Also, high safety can be realized with the use of naturally-occurring polymers.

### Example 4

The skin moisturizing effect of the solution comprising particles of Example 2 was evaluated. Eight of ten monitors answered that "the solutions exhibited greater moisturizing effects and imparted a fresh and non-sticky feeling to the skin." This indicates that a smaller particle diameter can realize higher transparency and superior extendibility when applied.

## Claims

1. An external dermatologic preparation which comprises protein nanoparticles containing an active ingredient and having an average particle size of 200 to 500 nm.

2. The external dermatologic preparation according to claim 1 wherein the protein nanoparticles contain an active ingredient in a weight that is 0.1% to 100% of the protein weight.

3. The external dermatologic preparation according to claim 1 wherein the active ingredient is at least one selected from the group consisting of ultraviolet absorber or moisturizing component.

4. The external dermatologic preparation according to claim 1 wherein the active ingredient is an ionic substance or a fat-soluble substance.

5. The external dermatologic preparation according to claim 1 wherein the protein is at least one selected from the group consisting of collagen, gelatin, acid-treated gelatin, albumin, ovalbumin, casein, transferrin, globulin, fibroin, fibrin, laminin, fibronectin, and vitronectin.

6. The external dermatologic preparation according to claim 1 wherein the protein is subjected to crosslinking treatment during and/or after nanoparticle formation.

7. The external dermatologic preparation according to claim 6 wherein crosslinking treatment is carried out by an enzyme.

8. The external dermatologic preparation according to claim 1 which comprises casein nanoparticles prepared by the following steps (a) to (c):
(a) mixing casein with a basic aqueous medium at pH of from 8 to less than 11;
(b) adding at least one active ingredient to the solution obtained in step (a); and
(c) injecting the solution obtained in step (b) into an aqueous medium at pH of 3.5 to 7.5:

9. The external dermatologic preparation according to claim 1 which comprises casein nanoparticles prepared by the following steps (a) to (c):
(a) mixing casein with a basic aqueous medium at pH of from 8 to less than 11;
(b) adding at least one active ingredient to the solution obtained in step (a); and
(c) lowering the pH of the solution obtained in step (b) to pH value which is different from the isoelectric point by 1 or more units.
